# EUROPEAN PATENT APPLICATION

(11) **EP 2 727 623 A1**
(43) Date of publication of application: **07.05.2014**
(21) Application number: 12191140.8
(22) Date of filing: 02.11.2012
(51) Int. Cl.: A61N 1/05, A61N 1/36

(54) **Electronics module with a cross-point switch matrix for brain applications**

(71) Applicant: Sapiens Steering Brain Stimulation B.V., 5656 AE Eindhoven (NL)
(72) Inventor: Tol, Jeroen Jacob Arnold, 5645 JG Eindhoven (NL); Martens, Hurbert Cécile Francois, 5611 ND Eindhoven (NL); Decré, Michel Marcel Jose, 5654 NC Eindhoven (NL); Budzelaar, Franciscus Paulus Maria, 5632 PL Eindhoven (NL)
(74) Representative: Prinz & Partner

(57) **Abstract**

The present application relates to an active electronics module (10) for a system for brain applications, whereby the active electronics module is connectable or connected to at least one main module (110) on the one side and on the other side connectable or connected to a lead (300) having a plurality of electrodes (132) at its distal end, whereby the active electronics module comprises at least one cross-point switch matrix means (20) configured such that at least one of the electrodes of the lead can be connected with one or more inputs and/or outputs of the main module.

## Description

The present invention relates to an active electronics module for a system for brain applications and to a system for brain applications.

Implantable neurostimulation devices have been used for the past 10 years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the 1.27 mm-diameter, 10-50 cm-long probes carry 4 annular electrodes at the distal end, that are connected to the Implantable Pulse Generator (IPG) using a 3.8 mm-diameter, 4 screw-contacts connector, by means of 2.8 mm-diameter extension cables. The proximal end of the probe has four concentric contacts that fit into the 4-contacts connector of the extension cable, thereby electrically connecting each electrode to the outputs of the IPG through a so-called "header".

Future systems will need more, smaller electrodes, in order to better control the delivery of electrical stimulation, because current stimulation causes mild to severe side-effects in about 30% of the patients. A larger number of electrodes means a larger number of contacts to the connector, which in turn calls for different connector technologies, because it cannot be expected from the neurosurgeon to tighten more than 10 individual screws for the more than 10 contacts. Also, the contact sizes need to be smaller, certainly in the case of cranial implants.

One drawback of existing neurostimulation is that existing neurostimulation devices can only address a small number electrodes, in particular 16 electrodes as a maximum, due to practical limitations on the number of connections that can be connected intra-operatively. Furthermore, due to the fact that existing implantable pulse generators should be modified as little as possible for cost reasons and for back-compatibility considerations, any improvements should leave the design of the implantable pulse generator modified as little as possible.

US 6,038,480 proposes an implantable controller means that are integrated in the lead at a site adjacent to the tissue to be stimulated with a main cable having at least one power conductor adapted to extend to a site adjacent said tissue, wherein the number of set power conductors is fewer than the number of said electrodes and where the cable connects to the implantable controller means. The system further comprises a source of data and a data conductor. The implantable controller of US 6,038,480 is further configured to establish an anode and cathode relationship between pairs of nonadjacent electrodes.

Although reducing the number of connections to be realized by the surgeon while increasing the number of addressable electrodes, the system according to US 6,038,480 has the disadvantage of increasing the size of the lead and may require significant changes to the standard procedure currently used to implant leads as well as the manufacturing procedures, as e.g. outlined in US 7,286,878 B2.

US 7,286,878 B2 and EP 1 446 189 B1 claim to solve this technical problem by an extension unit that is coupled between the implantable pulse generator and the implantable electrode array and is configured to electrically connect the output sources to a portion of the electrodes.

While having the advantage of not requiring to change the surgery for lead implantation and manufacturing procedures of leads, the systems according to US 7,286,878 B2 and EP 1 446 189 B1 do not at all address the problem of many connections that have to be made intra-operatively, a fact further stressed as an extension unit is also implanted between the electrode array and the pulse generator. Such a solution requires an additional device to be implanted and becomes prohibitive when the number of contacts exceeds 10 or more.

The system according to US 7,286,878 B2 has the further disadvantage that each switch is being connected between one output source and at least a portion of the electrodes to simultaneously trigger a plurality of electrodes. This significantly restricts the connection options, which is not desired.

Is therefore an object of the present invention to provide an active electronics module for an implant system and an implant system, in particular in that several electrodes of the lead of the implant system can be connected with the main module without having too many wires between the lead and the main module.

The above object is solved according to the present invention with an active electronics module for a deep brain stimulation system with the features of claim 1. Accordingly, an active electronics module for for a system for brain applications is provided, whereby the active electronics module is configured such that the active electronics module is connectable or connected to at least one main module on the one side and on the other side connectable or connected to a lead of the system for brain applications having a plurality of electrodes at its distal end, whereby the active electronics module comprises at least one cross-point switch matrix means configured such that at least one of the electrodes of the lead can be connected with one or more inputs and/or outputs of the main module of the system for brain applications.

The system for brain applications may be e.g. a system for neurostimulation and/or neurorecording. The system for neurostimulation and/or neurorecording may be preferably a system for deep brain stimulation.

The main module may preferably comprise or be embodied as a pulse generator and/or signal recording means, e.g. an implantable pulse generator unit, preferably an implantable pulse generator unit for a neuromodulation and/or neurorecording system with or without recording means for electrical signals.

The problem of having a lead with a high number of electrodes or sites at its distal end on the one hand, and a main module containing, for example, an implantable pulse generator (IPG) unit and neural recording facilities on the other, calls for a solution to randomly connect one or more sites with the main module.

This can be achieved according to the present invention by the application of an active electronics module (pre)connected to the multi-electrode lead, where this lead module at least contains a so called cross-point switch matrix means to connect the desired electrodes with one or more inputs and/or outputs of the main module of the neural implant. This essential step reduces the number of wires between lead and main module substantially and makes it feasible to apply this approach intra-operatively.

However, an active lead module introduces also extra wires, besides those needed for, for example, one or more stimulation channels, because of its need for power, communication and usually clocking. Still, one does not want to have too many wires between lead and main module, because only a small number of wires can be handled during surgery, while the connector pin count has to be reduced for reliability, size and cost reasons.

According to the present invention a new modular neural implant concept and an interface definition and protocol can be provided to minimize the number of wires between lead and main modules without sacrificing either functionality or integration of electronics in the lead itself. The foundation of this new modular concept is a (pre)connected lead module with a cross-point switch matrix means, which can preferably be realized as an (active) cross-point switch matrix. Advantageously, by means of the active electronics module according to the present invention several electrodes of the lead of the implant system can be connected with the main module without having too many wires between the lead and the main module.

In particular, the active electronics module comprises at least one rectifying means and/or at least one voltage converter means and/or at least one switch controlling means and/or at least one communication block means and/or at least one protection means. The rectifying means may be embodied as and/or comprising a rectifier. The voltage converter means may be embodied as and/or comprising a voltage converter. The switch controlling means may be embodied as and/or comprising a switch controller. The communication block means may be embodied as and/or comprising a communication block. The protection means may be e.g. embodied as a protection diode.

Furthermore, it is possible that the active electronics module comprises at least one first connection means configured such that the active electronics module can be connected to a pulse generator line being connectable to a pulse generator output and/or that the active electronics module comprises at least one second connection means configured such that the active electronics module can be connected to a data input line and/or output line and/or that the active electronics module comprises at least one third connection means configured such that the active electronics module can be connected to a power line and/or clock line and/or that the active electronics module comprises at least one fourth connection means configured such that the active electronics module can be connected to a connection line for battery ground.

The power line and/or clock line may be e.g. a combined power and clock line and may be used to transmit a power and a clock signal, which can be a bipolar square wave voltage. This same square wave can be used as a (reference) clock by the active module's electronics. The rectified square wave voltage can serve as the supply voltage for the active module's electronics. If the rectified voltage of the power and clock line is too low to supply the electronics directly, voltage boosting can be applied directly after rectification.

If corrosion and other unwanted irreversible electrochemical reactions at the connector terminals, applied to wire the pulse generator to the cross-point switch matrix means, are a concern, for example due to contact with saline body tissue, the average current on the power and clock line must be (nearly) zero, i.e. almost no leakage current. This in turn limits the allowable amplitude and frequency of the applied square wave.

Also, the data input line and/or output line may be e.g. embodied as a data input and output line, which is however not mandatory. The data input and output line may be used for communication between the pulse generator and the cross-point switch matrix means, including data communication to program the matrix to connect the required lead electrodes for field steering to the pulse generator output of choice of the pulse generator.

Both the pulse generator and the cross-point switch matrix means may be hermetically sealed and made of a conductive material, for example titanium, which can both be connected to battery ground, depending on the chosen grounding and leakage current strategy.

It is further possible that the active electronics module is configured such that at least two of the functions of the pulse generator line and/or the data input line and/or output line and/or the power line and/or clock line and/or the connection line for battery ground can be grouped and/or redistributed onto one or more lines, preferably onto at least one single line. Thereby, the advantage is achieved that the number of lines, in particular the number of physical wires can be reduced, since e.g. several functions can be grouped and redistributed onto one common line resulting in that e.g. only one physical wire is needed for the grouped functionality. Moreover, it is possible that e.g. in cases when the connection line for battery ground is redistributed, the connection line for battery ground can be replaced by at least some parts of a housing of the active electronics module and/or by at least one conductive means being in conductive contact with the active electronics module.

Beside the functions of the pulse generator line, the data input line and/or output line, the power line and/or clock line and/or the connection line for battery ground there may be more functions that may be grouped and/or redistributed onto one or more lines.

Additionally, it is possible that the active electronics module comprises an interface means having at least one communication protocol means configured such that at least two of the functions of the pulse generator line, the data input line and/or output line and/or the power line and/or clock line and/or the connection line for battery ground can be grouped and/or redistributed onto one or more lines, preferably onto at least one single line.

The interface means may be part of the at least one communication block means or may be embodied by the at least one communication block means.

It is possible that the rectifying means is arranged between the third connection means and the fourth connection means and/or that the rectifying means is configured such that an applied AC voltage can be rectified into a DC voltage, whereby preferably the DC voltage is a local supply voltage of the cross-point switch matrix means.

Moreover, the voltage converter means is configured such that a DC voltage can be boosted into a negative or positive supply voltage, which serves as a substrate voltage of a chip containing the cross-point switch matrix functions, whereby preferably the voltage converter means is a charge pump means, especially a voltage booster.

Additionally, it is possible that the DC voltage is the AC voltage rectified by the rectifying means and that the active electronics module comprises at least one connecting line configured such that the voltage converter means and the rectifying means are connected directly and/or indirectly.

Preferably, it is possible that the switch controlling means comprises at least one memory means, whereby the memory means is configured such that the state, in particular the on/off state, of switches of the cross-point switch matrix means is storable or stored in the memory means.

Further preferably, it is possible that each switch has a local floating supply in the form of a floating capacitor, which is recharged in a recharge cycle in a rhythm synchronized with the generated stimulation pulses that are applied by the system for brain applications, preferably applied to a patient connected to and/or being treated by the system for brain applications, whereby the switch controlling means is configured such that the recharge cycle is controlled by the switch controlling means. A synchronized rhythm may be the same rhythm as the stimulation or a rhythm, which recharges e.g. every 3^{rd}, 4^{th} etc. stimulation period.

Furthermore, the communication block means may be configured such that the communication block means is capable to monitor the data exchange between the main module and the cross-point switch matrix means.

Additionally, it is possible that the communication block means is configured such that the communication block means receives its clock via a connection means being connectable or connected with the at least one third connection means configured such that the active electronics module can be connected to a power line and/or clock line.

Moreover, the communication block means may be configured such that the communication block means is able to communicate internally with the switch controlling means, preferably to program the memory means of the switch controlling means, and/or that the communication block means is able to communicate internally with the voltage converter means, preferably to set and/or change the pump frequency and/or number of stages of the voltage converter means.

Preferably, it is possible that the communication block means is configured such that communication block means is able to communicate externally with the main module, preferably with the implantable pulse generator unit.

It is preferably possible that the protection means is a Schottky diode.

Additionally, it is possible that there are at least two protection diodes and/or that the protection means is configured and arranged such that if during start-up or any other situation the voltage converter means has not brought the substrate of a chip containing the cross-point switch matrix functions to its desired final negative and/or positive voltage yet before larger negative and/or positive voltages are applied either on the pulse generator output and/or power line and/or clock line and/or the data input line and/or output line, the substrate will be pulled down and/or up by the protection diode.

Furthermore, the present invention relates to an system for brain applications with the features of claim 15. Accordingly, an system for brain applications comprising at least one active electronics module according to any of the claims 1 to 14 is provided. Exemplarily the system for brain applications may be a medical implant system, whereby especially the implant system is a neural implant system, e.g. a neuromodulation and/or neurorecording system, preferably a deep brain stimulation system.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe neurostimulation system for deep brain stimulation (DBS) and its components;
- Fig. 3:: a schematical drawing of a probe system according to the present invention;
- Fig. 4:: a schematical drawing of a DBS system equipped with an active electronics module for a deep brain stimulation system;
- Fig. 5:: a further schematical drawing of a DBS system equipped with an active electronics module for a deep brain stimulation system shown in Fig. 4;
- Fig. 6:: a further schematical drawing of a DBS system equipped with an active electronics module for a deep brain stimulation system shown in Fig. 4 and 5;
- Fig. 7:: a schematical drawing of a further embodiment of a DBS system equipped with an active electronics module for a deep brain stimulation system;

- Fig. 8:: a schematical drawing of a further embodiment of a DBS system equipped with an active electronics module for a deep brain stimulation system; and
- Fig. 9:: a schematical drawing of a further embodiment of a DBS system equipped with an active electronics module for a deep brain stimulation system.

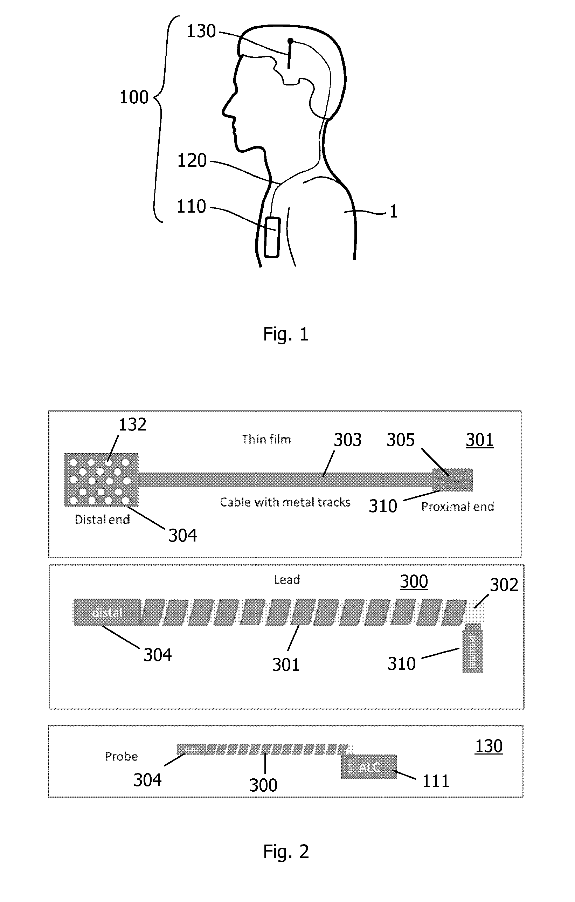

A possible embodiment of a neurostimulation system 100 for deep brain stimulation (DBS) is shown in Figure 1. The neurostimulation system 100 comprises at least a controller 110 or main module 110 that may be surgically implanted in the chest region of a patient 1, typically below the clavicle or in the abdominal region of a patient 1. The controller 110 can be adapted to supply the necessary voltage and/or current pulses. The typical DBS system 100 may further include an extension cable 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Connector (ALC) element 111 comprising electronic means to address electrodes 132 on the distal end 304 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 on the lead 300 is electrically connected to the ALC element 111. The ALC element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises of metal lines (not shown) to connect each distal electrodes 132 to a designated proximal contact 305.

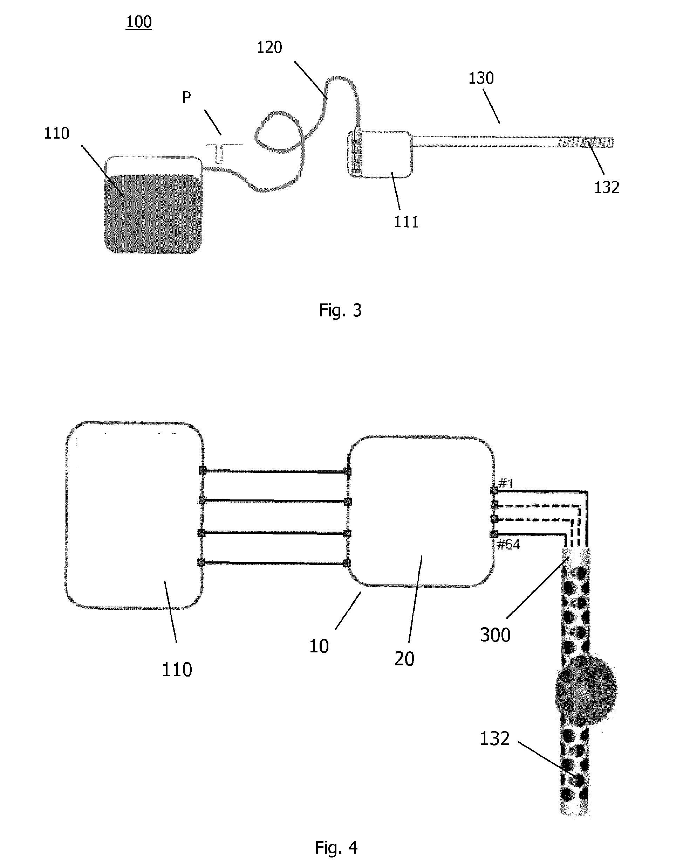

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 111. The controller 110 can be an implantable pulse generator (IPG) 110.

Figure 4 shows a schematical drawing of a DBS system 100 equipped with an active electronics module 10. A lead 300 with many electrodes 132, here 64 electrodes 132 at its distal end, is (pre)connected, during manufacturing of the lead, to a separate active electronic module 10 can containing at least cross-point switch matrix 20.

A main module 110, containing for example an IPG, is connected during surgery to this cross-point switch matrix can, for example via a screw connector, and together with the cross-point switch matrix/lead combination, a randomly selected group of one or more electrodes 132 can be connected to the main module 110, by programming the switch matrix, which effectively implements advanced field steering.

The separate cross-point switch matrix can, (pre)connected to the lead, with the ability to randomly connect any set of electrodes to the main module eliminates the drawbacks as mentioned in the above described prior art, which describe the current state-of-the-art to connect an IPG with a limited number of wires to address a multi-site lead.

Instead of transferring the stimulation signals for each electrode separately, the essential communication with the active lead module 10 consists of a transfer of stimulation pulses, a transfer of configuration data for the cross-point switch matrix and power for the active electronics inside the module.

Therefore, a reasonable upper limit for the number of wires needed to support these interface functions may be one wire/line for ground, one wire for power transfer, one wire/line for each pulse generator, two wires/lines for (unidirectional) data communication. Thus an implant with two pulse generators could be implemented with only 6 interface wires, irrespective of the number of electrodes that are driven.

A second main advantage of the invention is to reduce the number of wires further by grouping the individual functions of each physical wire and redistribute them over a smaller number of physical wires. For instance, power and data transfer can be combined on one physical wire. This implies somewhat more complex electronics but keeps the wire count low, even for a multi-channel interface. In the embodiments of this invention, several examples are described that combine several interface functions on a few wires to reduce the wire count.

To achieve this, the active electronics module may be configured such that at least two of the functions of the pulse generator line, the data input and output line and/or the power and clock line can be grouped and/or redistributed onto one or more lines, preferably onto at least one single line. Thereby, the advantage is achieved that the number of lines, in particular the number of physical wires can be reduced, since e.g. several functions can be grouped and redistributed onto one common line resulting in that e.g. only one physical wire is needed for the grouped functionality. Beside the functions of the pulse generator line, the data input and output line, the power and clock line and/or the connection line for battery ground there may be more functions that may be grouped and/or redistributed onto one or more lines. Additionally, it is possible that the active electronics module comprises an interface means having at least one communication protocol means configured such that at least two of the functions of the pulse generator line, the data input and output line and/or the power and clock line can be grouped and/or redistributed onto one or more lines, preferably onto at least one single line. The interface means may be part of the at least one communication block means or may be embodied by the at least one communication block means.

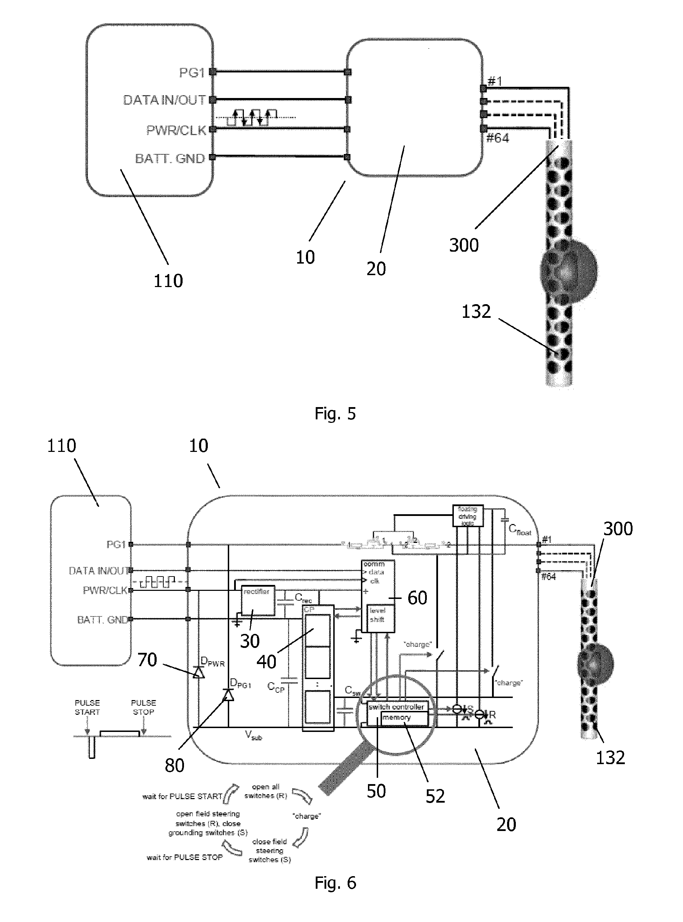

In a first implementation of the modular concept of a DBS system 100, as shown in detail in Figure 5, the IPG 110 has a single pulse generator output (PG1), a data channel (DATA IN/OUT), a combined power and clock line (PWR/CLK) and a connection for battery ground (BATT. GND), which are connected with the lead module 10 (active electronics module 10) with the cross-point switch matrix functionality. The lead module 10 is connected to the lead 300 with 64 electrodes 132.

The power (PWR) and clock (CLK) line may be used to transmit a power and clock signal, which can be a bipolar square wave voltage. This same square wave can be used inside the cross-point switch matrix means 20 (see also Figure 6) as reference clock. The rectified square wave voltage can serve as the supply voltage for the active module's electronics. If the rectified voltage of the power and clock line is too low to supply the electronics directly, voltage boosting can be applied directly after rectification.

If corrosion and other unwanted irreversible electrochemical reactions at the connector terminals, applied to wire the pulse generator to the cross-point switch matrix means, are a concern, for example due to contact with saline body tissue, the average current on the power and clock line must be (nearly) zero, i.e. almost no leakage current. This in turn limits the allowable amplitude and frequency of the applied square wave.

The DATA IN/OUT line is used for communication between IPG 110 and active module 10, including data communication to program the cross-point switch matrix 20 to connect the required lead electrodes 132 for field steering to the pulse generator output of IPG 110.

Both the cans of IPG 110 and cross-point switch matrix 20 are hermetically sealed and made of a conductive material, for example titanium, which can both be connected to battery ground (BATT. GND), depending on the chosen grounding and leakage current strategy.

The cross-point switch matrix functionality is contained in a hermetically sealed can implanted in the skull of a patient, and may, therefore, not be necessarily integrated as part of the lead 300 itself. The cross-point switch matrix 20 can is, however, pre-connected to the lead 300 during manufacturing. Also note again that the cross-point switch matrix 20 enables the connection of any random set of electrodes 132 to the IPG 110.

A high-level view of a cross-point switch matrix architecture is shown in Figure 6. Its basic component is the floating switch as shown in the top right part of Figure 6.

Although only a single floating switch is shown, connecting electrode 132 #1 of the lead 300 with the pulse generator output PG1 of the IPG 110, the other 63 electrodes 132, #2-64 in this example, can each individually be connected via a similar floating switch with the pulse generator output PG1 of the IPG 110. Every switch can individually be programmed to be conducting or not, and in this way, field steering can be implemented by appropriately programming the switch matrix.

The basic functionality of the switch matrix is shown in Figure 6 and going from left to right in the matrix can, the following building blocks and components are shown:

A rectifier 30 between the PWR/CLK line and battery ground BATT. GND is provided and rectifies the applied AC voltage (with an average current of zero) into a DC voltage across reservoir capacitor C_{rec} which can be an external component. This rectified voltage is the local supply voltage of the cross-point switch matrix can 20.

A charge pump (CP) 40 boosts this local supply voltage capacitively into a negative supply V_{sub} which serves as the substrate voltage of the chip which contains the cross-point switch matrix functions. Its voltage is lower than the most negative voltage which can appear across the lead's terminals when a negative stimulation pulse is applied. A capacitor C_{CP} between battery ground and the substrate is added to have a sufficiently stable negative supply rail.

A switch controller 50 with memory 52 controls the switch matrix 20, where the state (on/off) of every individual switch is stored in the controller's memory 52.

Every individual switch has a local floating supply in the form of a floating capacitor C_{float} which is (re)charged in the same rhythm as the stimulation pulses are applied to a patient 1.

A possible implementation of the (re)charge cycle of the switch controller can be as follows:

The controller 50 waits for the PULSE START signal which is given just before the IPG sends out a stimulation pulse as is also indicated in Figure 6. After the PULSE START signal is received all switches of the switch matrix 20 are opened i.e. made non-conducting. Next, the floating capacitors C_{float} of every individual switch is (re)charged during the "charge" state of the controller, which implies that the "charge" switches are temporarily closed and opened again. This (re)charge is taken from a capacitor C_{sw} which is most likely an external capacitor, because an on-chip capacitor C_{sw} implies it will be of the same order of magnitude of all the floating capacitors C_{float} of the switch matrix together to prevent a significant voltage drop across C_{sw} during the (re)charge phase and the charge pump 40 is most efficiently designed if it only needs to deliver the required (re)charge over the total available time i.e. the pulse stimulation period, typically the rate is 130 Hz. Moreover, the required charge needs to be delivered instantaneously. After the charging phase, the electrodes 132 needed to perform field steering are connected with the IPG 110. Those switches are closed until the PULSE STOP signal is received from the IPG 110 signalling the end of the stimulation pulse. Finally, the switches used during stimulation are opened again and all electrodes 132 are subsequently connected to battery ground i.e. BATT. GND. Those switches are not explicitly shown in Figure 6. This cycle may be repeated continuously by the switch controller 50 during stimulation.

A communication block 60 (comm block 60) takes care of data exchange between IPG 110 and the essential blocks in the switch matrix can 20. This digital block 60 receives its clock from the AC power line.

The communication block 60 communicates internally with both the switch controller 50, for example to program its internal memory, and with the charge pump 40, for example, to set its pump frequency and number of stages. Communication from these two blocks back to the communication unit 60 is also foreseen.

The communication block 60 also communicates externally with the IPG 110 via the DATA IN/OUT line. For example, on the rising edge of the PWR/CLK signal one or more bits can be transferred from IPG 110 to switch matrix can 20, while on a falling edge the IPG 110 can start to listen for a non-zero voltage on its DATA IN/OUT representing the transfer of one or more bits from the switch matrix can 20 to the IPG 110. It is possible that potentially communication between the communication block 60 and the rectifier 30 can also be added if required.

Furthermore, protection diodes 70 and 80 are provided, which are external Schottky diodes 70, 80 (DPWR and DPG1) between the negative supply rail V_{sub} and the PWR/CLK and PG1 lines, respectively, make sure that if during start-up, or any other situation, the charge pump 40 has not pumped the substrate to its desired final negative voltage yet before larger (negative) voltages are applied on either of the PWR/CLK and PG1 lines, the substrate will be pulled down by those protection diodes 70, 80.

As the naming already implies, the diodes 70, 80 are for protection purposes, preventing forward biasing the substrate junction isolation diodes, which in normal operation should never happen. Also, if data transmission across the DATA IN/OUT line is also done with negative voltages, an additional protection diode can be put on this line.

A different set of electrodes 132 can be stimulated during a stimulation period in a time multiplexed way with pulse generator 110, that is, the cross-point switch matrix 20 can be reprogrammed to connect a different set of electrodes 132 to the same pulse generator 110 as long as the reprogramming time of the matrix 20 is sufficiently short such that both the matrix 20 and pulse generator 110 are ready to deliver another stimulation pulse in time to, in this case, a different set of electrodes 132.

It is also possible to scavenge power from the stimulation pulses themselves to power the lead can electronics. This, however, is not further pursued in the embodiments described above and hereinafter, because it doesn't lead to a further reduction in interface wires.

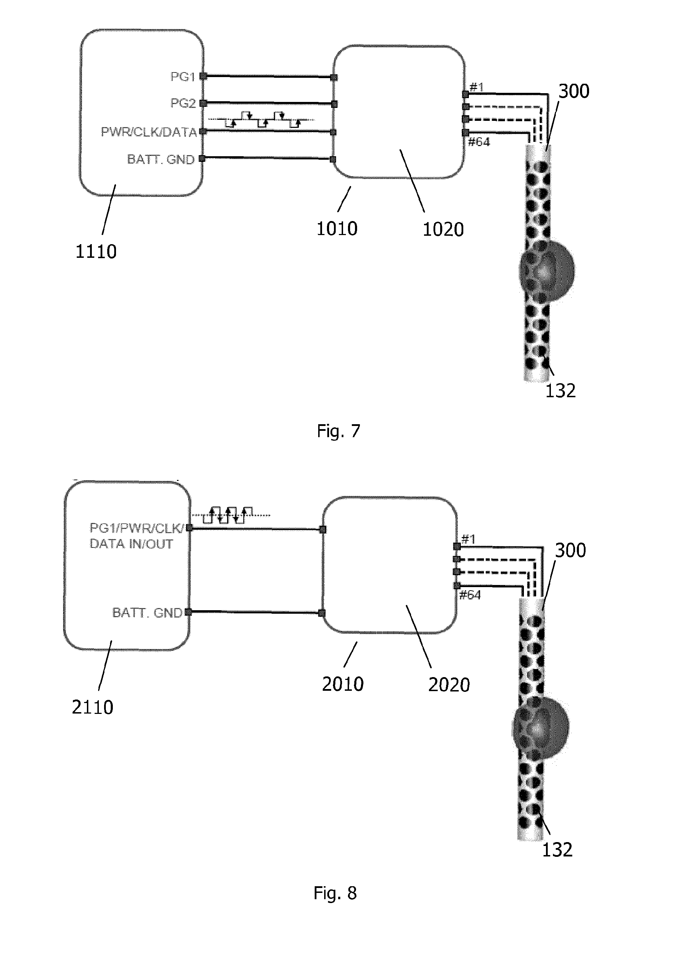

Another implementation of the modular concept is shown in Figure 7, which differs from the previous concept in the number of pulse generators, two instead of one, and the combination of both power and clock with data communication on a single wire between IPG 1110 and cross-point switch matrix 1020 in the lead module 1010. The IPG 1110 has two pulse generator outputs (PG1 and PG2) and a combined power, clock and data line (PWR/CLK/DATA). In this way, the number of wires between IPG 1110 and switch matrix 1020 is still limited to 4 wires which is identical to currently implanted DBS stimulators. The lead module 1010 is furthermore connected to a DBS lead 300 having 64 electrodes 132 (as already described above).

The architecture of the electronics inside the switch matrix 1020 can is the same as shown in Figure 6 except for the communication comm block 60 which doesn't have a separate data input anymore, while an additional set of floating switches has been added to the switch matrix to connect a random set of electrodes to the second pulse generator output IPG2 of the IPG 1110.

Again, the signal on the PWR/CLK/DATA line is such that potential chemical reactions at the connector terminals are prevented despite the fact that this same wire is now also used for data transmission. This combination of 3 different signals on a single wire can potentially be implemented as follows:

The alternating positive and negative pulses serve both for power transfer and switch can clock signal. A communication time slot is reserved between negative and positive pulses for data transmission. Adverse chemical reactions at the connector terminals are prevented if the chosen communication signal has (almost) no DC current content.

After the rising edge of the negative pulse the cross-point switch matrix can listens for a voltage change on its PWR/CLK/DATA line whose level and/or form is subsequently interpreted as one or more bits. After the falling edge of a positive pulse, this can be done the other way around: the switch matrix controls the PWR/CLK/DATA line while the IPG goes into listening mode.

Although Figure 7 suggests a PWR/CLK/DATA signal whose level is zero in between pulses, in reality, as just described above, one or more bits can be transferred between IPG and matrix by having a non-zero line voltage during the communication slot. Of course, no change can be chosen to represent a zero in a one bit protocol.

Other communication protocols are possible.

If the application allows addressing different electrode configurations in a time-multiplexed way, only a single, i.e. one pulse generator line is needed.

Another implementation of the modular concept is shown in Figure 8, which shows a duty-cycled neural stimulation enabling a two wires interface where the same wire is used for power and (bi)directional communication, including clocking, to the lead module 2010, which is connected to an IPG 2110 with a single pulse generator. The lead module 2010 is furthermore connected to a DBS lead 300 having 64 electrodes 132 (as already described above).

In neural stimulation, the stimulation pulses are duty-cycled, that is, only during a small portion of the total stimulation period, stimulation is actually applied. For example, a typical stimulation frequency is 130 Hz or a period of 7.7 ms of which only a couple of hundred microseconds are used for stimulation.

After a stimulation pulse has been applied, the remaining time in the stimulation period can be used for power and communication, including clocking, to the lead module. This leads to a two wires interface as shown in Figure 8.

Internally, (floating) high-voltage switches connect the PG1/PWR/CLK/DATA IN/OUT line to the power and communication module of the cross-point switch matrix 2020 can after a stimulation pulse has been given. The PULSE STOP signal can be reused for this. It can internally be generated by the active module's electronics via a stimulation pulse time-out timer with a pre-programmed value longer than the next stimulation pulse duration. The timer starts after the PULSE START signal has been received from the IPG.

Just before a new stimulation pulse, for which the PULSE START signal can be reused, the floating line switches are opened and their floating capacitors are (re)charged with all the other switches in the matrix simultaneously. Finally, the PG1/PWR/CLK/DATA IN/OUT line is connected to the original field steering switches to connect the desired electrodes to the pulse generator output of the IPG 2110.

The reservoir capacitor C_{rec} must provide power to the matrix can electronics when the actual stimulation takes place. The communication module must provide continuous clocking even when the reference clock is not present during the actual stimulation; for this standard clock (recovery) circuitry is known.

Again, if the application allows addressing different electrode configurations in a time-multiplexed way, only a single pulse generator is needed. This can be handled with the two wires interface a shown in Figure 8 as long as the application allows sufficient time for each signal on the PG1/PWR/CLK/DATA IN/OUT line so that the active electronics module can correctly operate. This includes the time needed to reprogram the switch matrix to address different sets of electrodes in time.

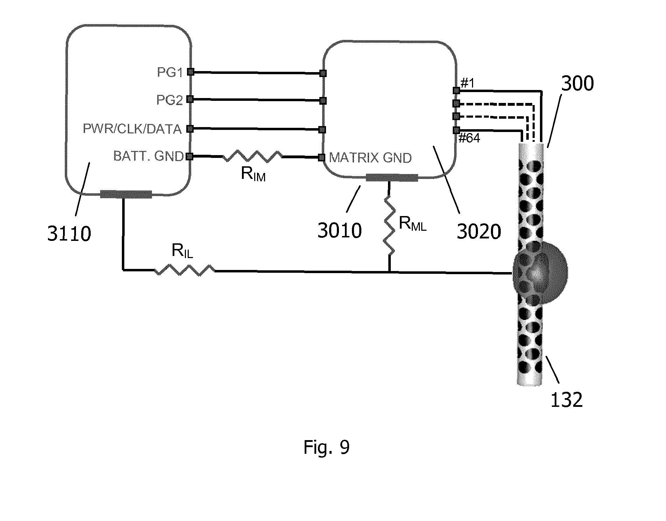

Another implementation of the modular concept is shown in Figure 9, which shows modular neural implant concept without a battery ground connection wire, in particular a modular neural implant concept with an IPG 3110 with two pulse generator outputs (PG1 and PG2) and a combined power, clock and data line (PWR/CLK/DATA) without a direct connection to battery ground of the IPG 3110.

In this embodiment, it is assumed that the IPC can 3110 has a direct, galvanic connection to battery ground (BATT. GND) of the IPG, while the switch matrix can 3020 is directly connected to its local ground (MATRIX GND) but not hard wired to the battery ground (BATT. GND) of the IPG.

Thus the local ground (MATRIX GND) of the switch matrix can is going to bounce up and down relative to the IPG's battery ground (BATT. GND) in the same rhythm as the stimulation pulses are applied to the brains.

The intensity of this local ground bounce depends on the relative resistance levels of RIM, RIL and RML, respectively, and therefore, the IPG 3110 and switch matrix cans 3020 should be positioned with respect to each other and the lead 300 with the 64 electrodes 132 in such a way that the voltage drop across RIM is minimized. In addition, the cross-point switch matrix electronics should be designed to be robust against ground bounce.

In the embodiment shown in Figure 9 it is tried to explicitly show that the casing of both the IPG 3110 and switch matrix 3020 is conductive and connected to their respective grounds i.e. BATT. GND and MATRIX GND, respectively.

It is thus possible in the implementations of the modular concept discussed above to remove the connection to battery ground between IPG 3110 and electronics implemented in the lead module 3010 as is shown in Figure 9 to save another wire between IPG 3110 and cross-point switch matrix can 3020 if the arising (matrix) ground bounce is acceptable.

The cross-point switch matrix 3020 starts to float with respect to battery ground once the direct connection to BATT. GND is removed due to the non-zero resistance of body tissue and the skull. It implies that not only the return current of the switch matrix itself flows back via the conductive skull and the body tissue but also (part of) the relatively large stimulation currents do. The possible return paths to BATT. GND of both stimulation currents as well as switch matrix currents are indicated by the resistances RIM, RIL and RML.

In the examples according to the present invention, which are described above, the lead can basically contained only cross-point switch matrix functionality with supporting electronics. However, it is possible to extend this with additional features. For example, it is possible to include neural recording amplifiers and (re)use the already available communication module to transport the digitized date to the main IPG module for further processing and/or wirelessly transmission to, for example, a receiver in the clinician's room when a patient comes over for a regular check-up.

## Claims

1. An active electronics module (10, 1010, 2010, 3010) for a system for brain applications (100), especially for a system for neurostimulation and/or neurorecording (100), whereby the active electronics module (10, 1010, 2010, 3010) is configured such that the active electronics module (10, 1010, 2010, 3010) is connectable or connected to at least one main module (110, 1110, 2110, 3110) on the one side and on the other side connectable or connected to a lead (300) of the system for brain applications (100) having a plurality of electrodes (132) at its distal end, whereby the active electronics module (10, 1010, 2010, 3010) comprises at least one cross-point switch matrix means (20, 1020, 2020, 3020) configured such that at least one of the electrodes (132) of the lead (300) can be connected with one or more inputs and/or outputs of the main module (110, 1110, 2110, 3110) of the system for brain applications (100), especially of the system for neurostimulation and/or neurorecording (100).

2. The active electronics module (10, 1010, 2010, 3010) according to claim 1, characterized inthat
the active electronics module (10, 1010, 2010, 3010) comprises at least one rectifying means (30) and/or at least one voltage converter means (40) and/or at least one switch controlling means (50) and/or at least one communication block means (60) and/or at least one protection means (70, 80), whereby the at least one protection means (70, 80) is e.g. a protection diode.

3. The active electronics module (10, 1010, 2010, 3010) according to claim 1 or 2,
characterized inthat
the active electronics module (10, 1010, 2010, 3010) comprises at least one first connection means configured such that the active electronics module (10, 1010, 2010, 3010) can be connected to a pulse generator line being connectable to a pulse generator output (PG1, PG2) and/or that the active electronics module (10, 1010, 2010, 3010) comprises at least one second connection means configured such that the active electronics module (10, 1010, 2010, 3010) can be connected to a data input line and/or output line (DATA IN/OUT) and/or that the active electronics module (10, 1010, 2010, 3010) comprises at least one third connection means configured such that the active electronics module can be connected to a power line and/or clock line (PWR/CLK) and/or that the active electronics module (10, 1010, 2010, 3010) comprises at least one fourth connection means configured such that the active electronics module (10, 1010, 2010, 3010) can be connected to a connection line for battery ground (BATT. GND).

4. The active electronics module (10, 1010, 2010, 3010) according to claim 3, characterized inthat
the active electronics module (10, 1010, 2010, 3010) is configured such that at least two of the functions of the pulse generator line and/or the data input line and/or output line (DATA IN/OUT) and/or the power line and/or clock line (PWR/CLK) and/or the connection line for battery ground (BATT. GND) can be grouped and/or redistributed onto one or more lines, preferably onto at least one single line, whereby e.g. when the connection line for battery ground (BATT. GND) is redistributed, the connection line for battery ground (BATT. GND) can be replaced by at least some parts of a housing of the active electronics module (10, 1010, 2010, 3010) and/or by at least one conductive means being in conductive contact with the active electronics module (10, 1010, 2010, 3010).

5. The active electronics module (10, 1010, 2010, 3010) according to claim 4, **characterized in that**
the active electronics module (10, 1010, 2010, 3010) comprises an interface means having at least one communication protocol means configured such that at least two of the functions of the pulse generator line and/or the data input line and/or output line (DATA IN/OUT) and/or the power line and/or clock line (PWR/CLK) and/or the connection line for battery ground (BATT. GND) can be grouped and/or redistributed onto one or more lines, preferably onto at least one single line.

6. The active electronics module (10, 1010, 2010, 3010) according to claim 5, **characterized in that**
the interface means is part of the at least one communication block means (60) or is embodied by the at least one communication block means (60).

7. The active electronics module (10, 1010, 2010, 3010) according to any of claims 3 to 6, especially according to claim 2 and one of claims 3 to 6, **characterized in that**
the rectifying means (30) is arranged between the third connection means and the fourth connection means and/or that the rectifying means (30) is configured such that an applied AC voltage can be rectified into a DC voltage, whereby preferably the DC voltage is a local supply voltage of the cross-point switch matrix means (20).

8. The active electronics module (10, 1010, 2010, 3010) according to any of claims 2 to 7,
**characterized in that**
the voltage converter means (40) is configured such that a DC voltage can be boosted into a negative or positive supply voltage, which serves as a substrate voltage of a chip containing the cross-point switch matrix functions, whereby preferably the voltage converter means (40) is a charge pump means, especially a voltage booster.

9. The active electronics module (10, 1010, 2010, 3010) according to claim 8, especially according to claim 7 and 8,
**characterized in that**
the DC voltage is the AC voltage rectified by the rectifying means (30) and that the active electronics module (10, 1010, 2010, 3010) comprises at least one connecting line configured such that the voltage converter means (40) and the rectifying means (30) are connected directly and/or indirectly.

10. The active electronics module (10, 1010, 2010, 3010) according to any of claims 2 to 9,
**characterized in that**
the switch controlling means (50) comprises at least one memory means (52), whereby the memory means (52) is configured such that the state, in particular the on/off state, of switches of the cross-point switch matrix means (20) is storable or stored in the memory means (52).

11. The active electronics module (10, 1010, 2010, 3010) according to claim 10,
**characterized in that**
the each switch has a local floating supply in the form of a floating capacitor, which is recharged in a recharge cycle in a rhythm synchronized with the generated stimulation pulses that are applied by the system for brain applications (100), preferably applied to a patient connected to and/or being treated by the system for brain applications (100), whereby the switch controlling means (50) is configured such that the recharge cycle is controlled by the switch controlling means (50).

12. The active electronics module (10, 1010, 2010, 3010) according to any of claims 2 to 11,
**characterized in that**
the communication block means (60) is configured such that the communication block means (60) is capable to monitor the data exchange between the main module (110, 1110, 2110, 3110) and the cross-point switch matrix means (20, 1020, 2020, 3020).

13. The active electronics module (10, 1010, 2010, 3010) according to claim 12, especially to any of claims 3 to 11 and claim 12,
**characterized in that**
the communication block means (60) is configured such that the communication block means (60) receives its clock via a connection means being connectable or connected with the at least one third connection means configured such that the active electronics module (10, 1010, 2010, 3010) can be connected to a power line and/or clock line (PWR/CLK) and/or that the communication block means (60) is configured such that the communication block means (60) is able to communicate internally with the switch controlling means (50), preferably to program the memory means (52) of the switch controlling means (50), and/or that the communication block means (60) is able to communicate internally with the voltage converter means (40), preferably to set and/or change the frequency and/or number of stages of the voltage converter means (40) and/or that the communication block means (60) is configured such that communication block means (60) is able to communicate externally with the main module (110, 1110, 2110, 3110), preferably with the implantable pulse generator unit (110, 1110, 2110, 3110).

14. The active electronics module (10, 1010, 2010, 3010) according to any of claims 2 to 12,
**characterized in that**
the protection means (70, 80) is a Schottky diode and/or that there are at least two protection diodes (70, 80) and/or that the protection means (70, 80) is configured and arranged such that if during start-up or any other situation the voltage converter means (40) has not brought the substrate of a chip containing the cross-point switch matrix functions to its desired final negative and/or positive voltage yet before larger negative and/or positive voltages are applied either on the pulse generator output (PG1, PG2) and/or the power line and/or clock line (PWR/CLK) and/or the data input line and/or output line (DATA IN/OUT), the substrate will be pulled down and/or up by the protection means (70, 80).

15. A system for brain applications (100) comprising at least one active electronics module (10, 1010, 2010, 3010) according to any of the preceding claims, whereby exemplarily the system for brain applications (100) is a medical implant system, whereby especially the implant system (100) is a neural implant system, e.g. a neuromodulation and/or neurorecording system, preferably a deep brain stimulation system.
